# EUROPEAN PATENT APPLICATION

(11) **EP 4 413 861 A1**
(43) Date of publication of application: **14.08.2024**
(21) Application number: 23305183.8
(22) Date of filing: 10.02.2023
(51) Int. Cl.: A01P 7/02, A01N 37/52, A01N 59/06

(54) **COMPOSITION FOR CONTROLLING MITE INFESTATION**

(71) Applicant: Veto-Pharma, 91120 Palaiseau (FR)
(72) Inventor: The designation of the inventor has not yet been filed
(74) Representative: Cabinet Beau de Loménie

(57) **Abstract**

The present invention relates to a parasiticide composition for maintaining honeybee colonies and controlling mite infestation therein, and its use as a medicament or as a parasiticide. The present invention further relates to a method of maintaining honeybee colonies and controlling mite infestation. The present invention further relates to devices comprising a parasiticide composition according to the invention.

## Description

### Technical field

The present invention relates to a parasiticide composition for maintaining honeybee colonies and controlling mite infestation therein. The present invention further relates to a method of maintaining honeybee colonies and controlling mite infestation. The present invention further relates to devices comprising a parasiticide composition according to the invention.

### Background of the invention

The Western honeybee (*Apis mellifera* L., Hymenoptera: Apidae) is a species of crucial economic, agricultural, and environmental importance. The last few decades have witnessed a significant reduction in honeybee populations. One of the foremost reasons for such reduction is attributed to mite infestation in honeybee colonies.

Honeybee hives and beekeeping boxes can be a suitable habitat for various mites (Acari), including nonparasitic, omnivorous, pollen-feeding species, and parasites. Among the different mite species associated with honeybees, *Varroa destructor, Acarapis woodi, Varroa jacobsoni* and *Tropilaelaps clareae* are economically significant pests infesting honeybee colonies, which is likely to lead to the destruction of the beekeeping industry.

The *Varroa* mite is currently the greatest biotic stressor of bees, both in its capacity as a parasite and by acting as a vector for viruses, including the Acute bee paralysis virus (ABPV), Kashmir Bee virus (KBV), Israeli acute paralysis virus (IAPV) and the Deformed Wing Virus. *Varroa destructor* is an obligate parasite which attacks the different development stages of *A. mellifera. Varroa* mites attack honeybee colonies as an ectoparasite of adult and developing bees, spreading disease, affecting honeybee colony health, and reducing their lifespan. *Varroa* along with their vectored viruses affect the immune response of honeybees, resulting in them being more susceptible to various disease-causing agents.

Traditional methods employed in *Varroa* mite control have been to hang plastic strips impregnated with chemical pesticides between the wax combs of beehives. However, repeated use of chemical pesticides is known to cause lethal effects on honeybee growth. Furthermore, mites are rapidly developing resistance to many of the commonly used treatments, which has prompted researchers to develop numerous alternative methods to prevent and treat *Varroa* mite infestations. These methods range from structural or mechanical changes to beehive units in apiaries, use of new pesticides and methods of administration that are valuable and effective alternatives to the standard treatments.

Amitraz (N'-(2,4-dimethylphenyl)-N-[(2,4-dimethylphenyl)iminomethyl]-N-methyl methanimidamide / CAS 33089-61-1) is a tertiary amino compound that is 1,3,5-triazapenta-1,4-diene substituted by a methyl group at position 3 and 2,4-dimethylphenyl groups at positions 1 and 5. It is one such synthetic compound with insecticide and acaricide properties used worldwide on both animals and crops to control pests. Amitraz is a formamide exhibiting both acaricidal and insecticidal activity and is frequently used by beekeepers to protect honeybee colonies against *Varroa destructor* mites. Therefore, there is a need to provide a parasiticide composition having good stability, ensuring good reproducibility of dosage, beekeeper safety and efficient activity.

In view of the potential value of amitraz against parasitic mites there is a long-lasting need in the art to develop a formulation with enhanced and sustained release activity of the active, improved characteristics of the formulation in terms of stability, and efficient inhibitory activity against *Varroa* mites.

The objective of the present invention is to provide a stable parasiticide composition to control mite infestation in honeybee colonies. Another objective of the present invention is to provide a method for controlling mite infestation in honeybee colonies and method for maintaining honeybee colonies.

Another objective of the present invention is to provide the use of a stable parasiticide composition to control mite infestation in honeybee colonies.

### Summary of the invention

### Main aspects of the invention

In an aspect, the present invention relates to a parasiticide composition for controlling mite infestation, such as for controlling mite infestation in honeybee colonies, comprising:
(a) an octopamine receptor agonist, and
(b) a metal oxide.

In another aspect, the present invention relates to a device adapted for use in apiculture comprising a parasiticide composition according to the invention.

In another aspect, the present invention relates to a method for controlling mite infestation in honeybee colonies comprising exposing the honeybee colonies to a parasiticide composition according to the invention.

In another aspect, the present invention relates to the use of a parasiticide composition according to the invention for controlling mite infestation in a honeybee colony.

In another aspect, the present invention relates to the use of a metal oxide for stabilizing an octopamine receptor agonist.

### Further aspects of the invention

In an aspect, the present invention relates to a parasiticide composition for maintaining honeybee colonies and controlling mite infestation therein comprising:
(a) an effective amount of an octopamine receptor agonist,
(b) a stabilizing amount of a metal oxide, and
(c) optionally, one or more acceptable excipients.

In an aspect, the present invention relates to a parasiticide composition for maintaining honeybee colonies and controlling mite infestation therein comprising:
(a) an effective amount of amitraz,
(b) a stabilizing amount of a metal oxide, and
(c) optionally, one or more excipients.

In an aspect, the present invention relates to a semi-solid parasiticide composition for maintaining honeybee colonies and controlling mite infestation therein comprising:
(a) an effective amount of an octopamine receptor agonist,
(b) a stabilizing amount of a metal oxide, and
(c) one or more excipients, for example one or more excipients suitable for obtaining a semi-solid parasiticide composition, such as one or more excipients suitable for obtaining a gel or a paste.

In an aspect, the present invention relates to a semi-solid parasiticide composition for maintaining honeybee colonies and controlling mite infestation therein comprising:
(a) an effective amount of amitraz,
(b) a stabilizing amount of a metal oxide, and
(c) one or more excipients suitable for obtaining a semi-solid parasiticide composition, such as one or more excipients suitable for obtaining a gel or a paste.

In another aspect, the present invention relates to a parasiticide semi-solid composition for maintaining honeybee colonies and controlling mite infestation therein comprising:
(a) an effective amount of amitraz,
(b) a stabilizing amount of a metal oxide, and
(c) one or more excipients suitable for obtaining a semi-solid parasiticide composition, such as one or more excipients suitable for obtaining a gel or a paste,
wherein % degradation of amitraz is reduced and the total amount of amitraz in the formulation remains constant after a duration of at least twelve months of storage at room temperature.

In an aspect, the present invention provides the use of a parasiticide composition for maintaining honeybee colonies and controlling mite infestation therein, said composition comprising an effective amount of amitraz, a stabilizing amount of a metal oxide, and eventually one or more excipients; wherein % degradation content of amitraz is reduced and the total amount of amitraz in the formulation remains constant after a duration of at least twelve months of storage at room temperature.

In another aspect, the present invention provides a method for maintaining honeybee colonies and/or preventing or controlling mite infestation in honeybee colonies in a brood chamber comprising applying in the vicinity of a honey-bee colony or in an artificial honey bee colony brood chamber, a parasiticide composition comprising amitraz, a stabilizing amount of a metal oxide and one or more excipients, for example one or more excipients suitable for obtaining a semi-solid parasiticide composition, such as one or more excipients suitable for obtaining a paste.

In another aspect, the present invention provides a method of maintaining honeybee colonies and controlling mite infestation comprising adapting a dosing gun to release a predetermined dosage of a parasiticide semi-solid composition comprising amitraz, a stabilizing amount of a metal oxide, and one or more excipients, wherein the dosing gun releases an amount ranging from 0.5 ml to 125 ml per dosing from a cartridge containing the parasiticide composition, wherein the predetermined dosage released is in an amount ranging from 0.1 ml to 10 ml, each time the trigger is activated.

In another aspect, the present invention provides a method of maintaining honeybee colonies and controlling mite infestation comprising adapting a dosing gun to release a predetermined dosage of a parasiticide semi-solid composition comprising amitraz a stabilizing amount of a metal oxide, and one or more excipients, wherein the dosing gun releases is an amount ranging from 0.5 ml to 125 ml per dosing from a cartridge containing the parasiticide composition, wherein the predetermined dosage released is in an amount ranging from 1 ml to 3 ml, each time the trigger is activated.

### Detailed description of the invention

The following description is provided to assist in a comprehensive understanding of exemplary embodiments of the invention. It includes various specific details to assist in that understanding but these are to be regarded as merely exemplary.

Accordingly, those of ordinary skill in the art will recognize that various changes and modifications of the embodiments described herein can be made without departing from the scope of the invention. In addition, descriptions of well-known functions and constructions are omitted for clarity and conciseness.

The terms used in the following description and claims are not limited to the bibliographical meanings but are merely used by the inventor to enable a clear and consistent understanding of the invention. Accordingly, it should be apparent to those skilled in the art that the following description of exemplary embodiments of the present disclosure are provided for illustration purpose only and not for limiting the scope of the invention as defined by the appended claims and their equivalents.

For the purposes of the following detailed description, it is to be understood that the invention may assume various alternative variations and step sequences, except where expressly specified to the contrary. Moreover, other than in any operating examples, or where otherwise indicated, all numbers expressing, for example, quantities of materials/ingredients used in the specification are to be understood as being modified in all instances by the term "about".

Thus, before describing the present invention in detail, it is to be understood that this invention is not limited to particularly exemplified systems or process parameters that may of course, vary. It is also to be understood that the terminology used herein is for the purpose of describing embodiments of the invention only and is not intended to limit the scope of the invention in any manner. The use of examples anywhere in this specification including examples of any terms discussed herein is illustrative only, and in no way limits the scope and meaning of the invention or of any exemplified term.

It must be noted that, as used in this specification, the singular forms "a," "an" and "the" include plural referents unless the content clearly dictates otherwise. The terms "preferred" and "preferably" refer to embodiments of the invention that may afford certain benefits, under certain circumstances.

As used herein, the terms "comprising" "including," "having," "containing," "involving," and the like are to be understood to be open-ended, i.e., to mean including but not limited to.

### Definitions

The term 'semi-solid composition' refers to a composition usually solid at room temperature which can flows under mechanical stress. It includes compositions such as for example pastes, gels, creams, ointments. The consistency of semi-solid composition allows the appropriate diffusion of active ingredients such as a parasiticide drug substance.

The term "excipient suitable for obtaining a semi-solid composition" designates one or more ingredients able to give the adapted consistency to form a stable semi-solid composition. The skilled person will select the appropriate excipients for their capacity to give the adapted consistency to the said semi-solid composition. Examples of excipients suitable for obtaining a semi-solid composition are indicated hereafter:
- waxes, including waxes from natural origin (such as carnauba wax or, beeswax, (hydrogenated) vegetable oils) and synthetic waxes (such as petrolatum, including liquid and solid parrafin and their mixtures, ethylene or propylene oxide polymers),
- gelling or thickening agents which can have a polymeric structure including natural polymers (such as cellulose derivatives, xanthane gum, guar gum, carob gum, alginic acid derivatives, carrageenan) or synthetic polymers (such as styrene polymers, acrylic polymers, vinylic polymers),
- Non soluble compounds such as metal carbonates or oxide able to give consistency to a liquid after homogeneous dispersion by increasing the number of solid particles in the composition.

The term "parasiticide" refers to the ability of a substance to decrease or inhibit the growth of a parasite.

Ectoparasitic infection or infestations suitable for treatment by the method of the invention include fleas, ticks, lice, flies and mites, such as Varroa.

The terms "apiary" or "bee yard" herein refers to colonies, brood boxes, beehive units, and other equipment assembled in one location for beekeeping operations.

The terms 'bee-hive unit' or 'brood unit' or 'brood box' as used herein refers to a bee-hive having a brood-chamber located at its lower central portion of the unit, having further chambers above and partially at the side of the brood-chamber and separated therefrom by frames having a series of slots therein, and the chambers one at each lower side portion of the hive and partially under the brood-chamber, the partitions forming the walls of said chambers being formed each with a series of slots to permit free access of the bees from one portion of the hive to another.

To "control" or "controlling" mites means to inhibit, and/or supress the ability of mites to grow and/or reproduce, or to limit fungus-related damage or loss in crop plants or denotes control and prevention of a disease. Controlling effects include all deviation from natural development, for example: killing, retardation, decrease of the mite causing disease.

The term "locus" as used herein shall denote the vicinity of beehive in which control of the spread of an ectoparasite is desired. The locus includes the vicinity of beehive colonies, brood boxes, beehive units, wherein the mites or specifically Varroa mites has either emerged, initiated infestation or is most likely to emerge or is yet to emerge.

As described herein, "parasiticidal amount" is an amount of active ingredient causing an adversely modifying effect including deviations from natural development, killing, regulation, desiccation, retardation, and the like of the parasite.

The term "strip" according to the invention is made by extruding, cutting or braiding a flexible material. The flexible material can for example be a plastic material, a rubber, a fabric, a resin or polyholoside fibers. The strip can come in different shapes. Preferably, the strip is adapted for use in apiculture, that is to say a non-circular strip, preferably a flat strip, having a size, a rigidity and a shape adapted so that the strip can be slip between the frames of a hive. The strip can also be placed on the top of a frame or at the hive entrance.

Each of the aspects described above may have one or more embodiments.

Each of the embodiments described hereinafter may apply to one or all of the aspects described hereinabove. These embodiments are intended to be read as being preferred features of one or all aspects described hereinabove. Each of the embodiments described hereinafter applies to each of the aspects described hereinabove individually.

The present inventors have successfully provided a stable parasiticide composition comprising an octopamine receptor agonist, such as amitraz, wherein the % degradation products of the octopamine receptor agonist have been reduced and the total amount of the octopamine receptor agonist retained in the formulation remains constant after a time duration of at least twelve months at room temperature. The % bee mortality was also drastically reduced using the parasiticide composition of the present invention.

Accordingly, the present inventors have unexpectedly found the most appropriate amount of metal oxide, such as calcium oxide, to be added to a parasiticide composition comprising an octopamine receptor agonist, such as amitraz, which was found to be sufficient to achieve efficient octopamine receptor agonist dosing results and reduced octopamine receptor agonist degradation products.

The parasiticide composition of the invention in a semi-solid form has significant advantages over liquid form. Semi-solid compositions require no dilution and have improved handling characteristics, thereby reducing the hazards associated with handling liquid composition of octopamine receptor agonist, such as amitraz, which can be corrosive and spills and splashes easily. Additionally, the use of a semi-solid composition can help reduce contact between the active ingredient and the beekeepers. Additionally, the octopamine receptor agonist in a semi-solid composition is rapidly available for treating bees.

The parasiticide composition of the invention in a solid form may have a slow-release property, thus reducing the number of applications necessary to achieve adequate mite control. By controlling the release of the octopamine receptor agonist in a parasiticide solid composition, a reduced bee mortality is observed, and the longer exposure time of the mites with the parasiticide composition assures a higher miticide rate.

### Parasiticide composition of the invention

One object of the present invention provides a parasiticide composition for controlling mite infestation, such as for controlling mite infestation in honeybee colonies, comprising:
(a) an octopamine receptor agonist, and
(b) a metal oxide.

In an embodiment, the parasiticide composition of the invention comprises:
(a) an effective amount of an octopamine receptor agonist, and
(b) a stabilizing amount of a metal oxide.

In some embodiments, the octopamine receptor agonist is an octopamine receptor agonist of formula (I) disclosed in WO2021/116631A1, incorporated herein by reference, said formula (I) is: 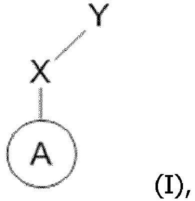 wherein:
A is selected from the group (II) or (III): 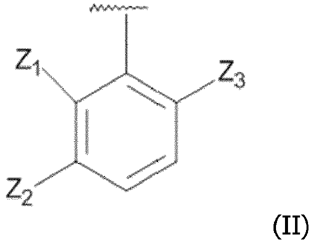 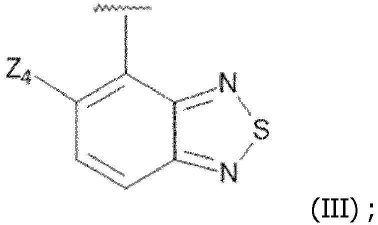
X is NH, CH2 or CH-CH3;
Y is a 5-bonded heterocycle comprising at least one nitrogen atom;
Z1 is a halogen, H or CH3;
Z2 is a halogen, H or CH3;
Z3 is a halogen, H or CH3;
Z4 is a halogen, H or CH3.

Advantageously, Y is the following group (IV) or (V):

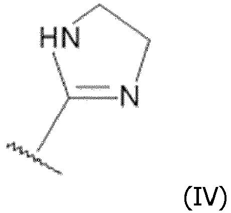

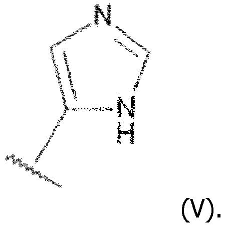

Advantageously, the compound of formula (I) is: 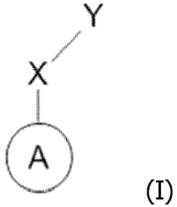 wherein:
- A is the group (II), X is NH, Y is the group (IV), Z1 is a halogen, Z2 is H and Z3 is a halogen,
- A is the group (II), X is CH2 or CH-CH3, Y is the group (V), Z1 is CH3, Z2 is CH3 and Z3 is H, or
- A is the group (III), X is NH, Y is the group (IV) and Z4 is a halogen.

Advantageously, the allogen is selected from F, Cl or Br.

In some embodiments, the compound of formula (I) is selected from detomidine (CAS number: 76631-46-4), medetomidine (D) (that is to say dexmedetomidine - CAS number: 113775-47-6), medetomidine (L) (that is to say levomedetomidine - CAS number: 119717-21-4), romifidine (CAS number: 65896-16-4), clonidine (CAS number: 4205-90-7), tizanidine (CAS number: 51322-75-9) or a mixture of two or more of these compounds. It may for example be a racemic mixture, for example the racemate of medetomidine (CAS number: 86347-14-0).

In some preferred embodiments, the octopamine receptor agonist is amitraz (CAS 33089-61-1).

In some embodiments, the metal oxide is selected from (i) oxides of alkali metals, such as sodium oxide and potassium oxide or (ii) alkaline earth metals, such as calcium oxide, barium oxide and magnesium oxide. Preferably, the metal oxide is calcium oxide.

In some preferred embodiments, the octopamine receptor agonist is amitraz and the metal oxide is calcium oxide.

In some embodiments, the parasiticide composition of the invention is (i) in a liquid form, such as an emulsion, a suspension or an oil dispersion (OD); (ii) in a semi-solid form, such as a gel or a paste; or (iii) in a solid form, such as an extruded composition, water dispersible granules (WDG), wettable powders (WP), or dusts.

Preferably, the parasiticide liquid composition comprises one or more excipients that are suitable for obtaining a liquid composition.

Preferably, the parasiticide semi-solid composition comprises one or more excipients that are suitable for obtaining a semi-solid composition, as detailed below.

Preferably, the parasiticide solid composition comprises one or more excipients that are suitable for obtaining a solid composition. The one or more excipients that are suitable for obtaining a solid composition comprise synthetic polymers, such as ethylene-vinyl acetate (EVA) or polyvinylchloride (PVC), or natural polymers, such as starch or modified starch. Preferably, the one or more excipients are suitable for obtaining a solid composition by extrusion. Extrusion required heating the composition at a high temperature, generally above 100°C, which can degrade the octopamine receptor agonist. The inventors have shown that an octopamine receptor agonist is particularly stable in a solid compositions prepared by extrusion comprising a metal oxide compared to a solid composition lacking a metal oxide.

In some embodiments, the metal oxide is in an amount sufficient to stabilize the octopamine receptor agonist in the composition.

In some embodiments, the percentage of degradation of the octopamine receptor agonist is reduced and the total amount of the octopamine receptor agonist in the composition remains constant after at least twelve months of storage at room temperature.

In some embodiments, the percentage of degradation of the octopamine receptor agonist is reduced and the total amount of the octopamine receptor agonist in the composition remains constant after at least twelve months of storage at 30°C. In some other embodiment, the percentage of degradation of the octopamine receptor agonist is reduced and the total amount of the octopamine receptor agonist in the composition remains constant after at least six months of storage at 40°C.

In some embodiments, the octopamine receptor agonist is in a concentration ranging from 0.1% to 20% w/w of the composition, such as from 0.5% to 10% w/w of the composition, such as from 1% to 5% w/w of the composition, such as from 1% to 3% w/w of the composition, such as 2% w/w of the composition.

In some embodiments, the metal oxide is in a concentration ranging from 1% to 20% w/w of the composition, such as from 5% to 15% w/w of the composition, such as from 5% to 10% w/w of the composition, such as 9% w/w of the composition.

In some embodiments, the composition further comprises one or more excipients, for example:
- A wax;
- A gelling or thickening agent; and/or.
- Non-soluble compounds.

In some embodiments, the wax includes (i) waxes from natural origin, such as carnauba wax or beeswax, (hydrogenated) vegetable oils; and/or (ii) synthetic waxes, such as petrolatum, preferably liquid and/or solid parraffin, ethylene oxide polymers (also known as "macrogols"), propylene oxide polymers or ethylene propylene oxide copolymers (also known as "macrogols derivatives").

In some embodiments, the gelling or thickening agent has a polymeric structure. The gelling or thickening agent includes (i) natural polymers, such as cellulose derivatives, xanthane gum, guar gum, carob gum, alginic acid derivatives, carrageenan and (ii) synthetic polymers, such as styrene polymers, acrylic polymers, vinylic polymers.

In some embodiments, non-soluble compounds are metal carbonates or oxide able to give consistency to a liquid after homogeneous dispersion by increasing the number of solid particles in the composition.

The one or more excipients may be food acceptable excipients and/or pharmaceutically acceptable excipients. Preferably, the one or more excipients are suitable for treating honeybees.

In some embodiments, the parasiticide composition of the invention further comprises one or more excipients, in a concentration ranging from 30% to 95% w/w of the composition, for example from 50% to 90% w/w of the composition, for example from 60% to 90% w/w of the composition, for example 88%.

In some embodiments, the parasiticide composition of the invention is a semi-solid composition, such as a gel or a paste, wherein the water content in the composition is at least <2% w/w (less than 2% w/w) of the composition, such as <1 % w/w (less than 1% w/w) of the composition, such as <0.5 % w/w (less than 0.5% w/w) of the composition.

In some embodiments, the parasiticide composition of the invention comprises:
- an effective amount of amitraz in a concentration ranging from 0.1% to 20% w/w of the composition,
- a stabilizing amount of calcium oxide in a concentration ranging from 1% to 20% w/w of the composition, and
- an excipient in a concentration ranging from 30% to 95% w/w of the composition.

In some embodiments, the parasiticide composition of the invention further comprises an excipient selected from the group comprising a carrier, a solvent, an emulsifier, a penetration enhancer, a preservative and an emollient, for example an antioxidant.

In some embodiments, the parasiticide composition is an acaricide composition, such as a varroacide composition.

In some embodiments, the parasiticide composition of the invention comprises:
(a) amitraz in a concentration of about 2.0% w/w of the composition;
(b) Calcium oxide in a concentration of about 9.5% w/w of the composition; and
(c) Petrolatum, preferably paraffin, in a concentration of about 88.5% w/w of the composition.

In a preferred embodiment, calcium oxide is CAS n°1305-78-8 and/or paraffin is CAS n°8009-03-8.

The following objects also define parasiticide compositions according to the invention.

The present invention provides a parasiticide composition for maintaining honeybee colonies and controlling mite infestation comprising:
(a) an effective amount of amitraz, and
(b) a stabilizing amount of a metal oxide
(c) optionally one or more excipients.

In an embodiment, the present invention provides a parasiticide semi-solid composition for maintaining honeybee colonies and controlling mite infestation comprising:
(a) an effective amount of amitraz, and
(b) a stabilizing amount of a metal oxide,
(c) optionally one or more excipients.

In some embodiments, the semi-solid composition is a paste or a gel.

In an embodiment, the parasiticide composition of the invention comprises amitraz in a concentration ranging from 0.1% to 20% w/w of the composition.

In another embodiment, the parasiticide composition of the invention comprises amitraz in a concentration ranging from 0.5% to 10% w/w of the composition.

In yet another embodiment, the parasiticide composition of the invention comprises amitraz in a concentration ranging from 1% to 5% w/w of the composition.

In a preferred embodiment, the parasiticide composition of the invention comprises amitraz in a concentration ranging from 1% to 3% w/w of the composition.

In a preferred embodiment, the parasiticide composition of the invention comprises amitraz in a concentration of 2% w/w of the composition.

In an embodiment, the parasiticide composition of the invention comprises metal oxide selected from oxides of alkali metals or alkaline earth metals.

In an embodiment, the metal oxide is an oxide of an alkali metal selected from the group comprising sodium and potassium.

In an embodiment, the metal oxide is an oxide of an alkaline earth metal selected from the group comprising calcium, barium, aluminium and magnesium. In a preferred embodiment, the metal oxide is calcium oxide.

In an embodiment, the parasiticide composition comprises a metal oxide in a concentration ranging from 1% to 20% w/w of the composition.

In an embodiment, the parasiticide composition comprises a metal oxide in a concentration ranging from 5% to 15% w/w of the composition.

In an embodiment, the parasiticide composition comprises calcium oxide in a concentration ranging from 1% to 20% w/w of the composition.

In an embodiment, the parasiticide composition comprises calcium oxide in a concentration ranging from 5% to 15% w/w of the composition.

In an embodiment, the parasiticide composition comprises calcium oxide in a concentration ranging from 5% to 10% w/w of the composition.

In an embodiment, the parasiticide composition comprises calcium oxide is in a concentration of 9% w/w of the composition.

In an embodiment, the parasiticide composition comprises one or more excipients, preferably one or more excipients designated as Generally Recognised As Safe (GRAS).

In an embodiment, the one or more excipients comprise petrolatum, such as petrolatum selected from the group comprising liquid paraffin and/or solid paraffin.

In an embodiment, the one or more excipients are in a concentration ranging from 30% to 95% w/w of the composition.

In an embodiment, the one or more excipients are in a concentration ranging from 50% to 90% w/w of the composition.

In an embodiment, the one or more excipients are in a concentration ranging from 60% to 90% w/w of the composition.

In an embodiment, the one or more excipients are in a concentration of 88% w/w of the composition.

In an embodiment, the one or more excipients are suitable for obtaining a semi-solid composition, such as a gel or a paste.

In an embodiment, the present invention provides a semi-solid parasiticide composition for maintaining honeybee colonies and controlling mite infestation comprising;
(a) an effective amount of amitraz in a concentration ranging from 0.1% to 20% w/w,
(b) a stabilizing amount of calcium oxide in a concentration ranging from 1% to 20% w/w, and
(c) one or more excipients in a concentration ranging from 30% to 95% w/w;
each component by weight of the total composition.

In an embodiment, the present invention provides a semi-solid parasiticide composition for maintaining honeybee colonies and controlling mite infestation comprising;
(a) an effective amount of amitraz in a concentration ranging from 1% to 5% w/w,
(b) a stabilizing amount of calcium oxide in a concentration ranging from 5% to 10% w/w, and
(c) petrolatum as an excipient in a concentration ranging from 50% to 90% w/w;
each component by weight of the composition.

In some embodiments, the semi-solid parasiticide composition is a paste or a gel. For example, the water content in a gel composition is at least <2% w/w (less than 2% w/w), such as at least < 1% w/w, for example at least < 0,5% w/w.

In an embodiment, the present invention provides a parasiticide semi-solid composition for maintaining honeybee colonies and controlling mite infestation comprising;
(a) an effective amount of amitraz,
(b) a stabilizing amount of a metal oxide, and
(c) one or more acceptable excipients suitable for obtaining a semi-solid composition.

In an embodiment, the present invention provides a parasiticide semi-solid composition for maintaining honeybee colonies and controlling mite infestation comprising;
(a) an effective amount of amitraz,
(b) a stabilizing amount of a metal oxide, and
(c) one or more acceptable excipients suitable for obtaining a semi-solid composition
wherein degradation content of amitraz is reduced and the total amount of amitraz in the formulation remains constant after a duration of at least twelve months of storage at room temperature.

In an embodiment, the present invention provides a parasiticide semi-solid composition for maintaining honeybee colonies and controlling mite infestation comprising;
(a) an effective amount of amitraz,
(b) a stabilizing amount of calcium oxide, and
(c) petrolatum;
wherein degradation content of amitraz is reduced and the total amount of amitraz in the composition remains constant after a duration of at least twelve months of storage at room temperature.

In an embodiment, the present invention provides a parasiticide semi-solid composition for maintaining honeybee colonies and controlling mite infestation comprising;
(a) an effective amount of amitraz in a concentration ranging from 0.1% to 20% w/w,
(b) a stabilizing amount of calcium oxide in a concentration ranging from 1% to 20% w/w, and
(c) petrolatum in a concentration ranging from 30% to 95% w/w; each component by weight of the composition;
wherein degradation content of amitraz is reduced and the total amount of amitraz in the composition remains constant after a duration of at least twelve months of storage at room temperature.

In an embodiment, the present invention provides a parasiticide semi-solid composition for maintaining honeybee colonies and controlling mite infestation comprising;
(a) an effective amount of amitraz in a concentration ranging from 1% to 5% w/w,
(b) a stabilizing amount of calcium oxide in a concentration ranging from 5% to 10% w/w, and
(c) petrolatum in a concentration ranging from 50% to 90% w/w; each component by weight of the composition;
wherein degradation content of amitraz is reduced and the total amount of amitraz in the composition remains constant after a duration of at least twelve months of storage at room temperature.

### Medical use of the parasiticide composition of the invention

Another object of the present invention provides a composition according to the invention, as described above, for use as a medicament. For example, the invention provides a composition according to the invention for use as a parasiticide.

The parasiticide composition of the invention can be extended for use as topical administrations in veterinary applications as well. Examples of topical administrations suitable for use in the invention include spot-on, pour-on, dip, spray, wash, shampoo, foam, gel, lotion, powder or any of the conventional means of topically applying a liquid veterinary composition. The topical mode or administration will vary with the species and size of the host animal (Bee, dog, etc.).

### Devices comprising the parasiticide composition of the invention

Another object of the present invention is a device adapted for use in apiculture comprising a parasiticide composition according to the invention. The device may be a strip, a brood chamber, a hive, a dosing gun or a kit of parts.

According to an embodiment, the various components of the stable parasiticide composition of the invention may be used individually or already partially or completely mixed with one another to prepare the said composition. It is also possible for the component to be separately packaged and used as a component in a kit of parts.

In one embodiment, the kits may include one or more of the components that are used to prepare a composition according to the invention. For examples, the kits may include the octopamine receptor agonist, the metal oxide, and eventually the excipient, as defined above. One or more of the components may already be combined together or pre-formulated.

According to an embodiment of the present disclosure, a kit comprising a stable parasiticide gel formulation is provided. The kit comprises a plurality of components, including at least one of the ingredients of the stable parasiticide composition of the present disclosure.

In one embodiment of the present disclosure, the kit includes at least one, or all of components needed to prepare the stable parasiticide composition. For example, the kit may include amitraz, the metal oxide and the excipient.

One or more of the components may already be combined together or pre-formulated. In those embodiments where more than two components are provided in a kit, the components may already be combined and as such are packaged in a single container such as a vial, bottle, can, pouch, bag and/or canister.

In a preferred embodiment, the device comprises a ready-to-use parasiticide composition according to the invention.

In some embodiments, the device is selected from patties, pastes, plastic porous strips, extruded strips, other strip materials, pads, powders, etc.

In some embodiments, the device is a porous support impregnated with the composition of the invention in a liquid or semi-solid form, for example a cellulosic porous support (e.g. cellulose) or a wood support.

### Method and use of the composition according to the invention

Another object of the present invention is a method for controlling mite infestation in honeybee colonies comprising exposing the honeybee colonies to a parasiticide composition according to the invention.

Another object of the present invention is the use of a parasiticide composition according to the invention for controlling mite infestation in honeybee colonies.

In some embodiments, the parasiticide composition reduces or prevents mite infestation.

In some embodiments, the mite is Varroa.

In some embodiments, the parasiticide composition is applied to a hive, preferably the composition is applied to a brood chamber.

In some embodiments, the composition is applied in an amount from 0.1 ml to 30 ml.

In an embodiment, the present invention provides a method for controlling mite infestation in a brood chamber of honeybee colonies comprising applying the parasiticide composition on separate supports introduced in the said brood chamber.

In an embodiment, the separate supports are wooden supports.

In an embodiment, the present invention provides a method of controlling mite infestation in a brood chamber in brood free honeybee colonies or when the brood is present.

In an embodiment, the present invention provides a method of controlling mite infestation in a brood chamber comprising adapting a dosing gun to release a predetermined dosage of a semi-solid parasiticide composition comprising amitraz, wherein the predetermined dosage released is in an amount ranging from 0.1 ml to 10 ml, each time the trigger is activated.

In an embodiment, the present invention provides a method of controlling mite infestation in a brood chamber comprising adapting a dosing gun to release a predetermined dosage of a parasiticide composition, wherein the predetermined dosage released is in an amount ranging from 0.1 ml to 6 ml, each time the trigger is activated.

In an embodiment, the present invention provides a method of controlling mite infestation in a brood chamber comprising adapting a dosing gun to release a predetermined dosage of a parasiticide composition of the invention, preferably wherein the predetermined dosage released is in an amount ranging from 0.1 ml to 30 ml, each time the trigger is activated.

In an embodiment, the present invention provides a method of controlling mite infestation in a brood chamber comprising adapting a dosing gun to release a predetermined dosage of a parasiticide composition of the invention, wherein the predetermined dosage released is in an amount ranging from 0.1 ml to 10 ml, each time the trigger is activated.

In another embodiment, the present invention provides a method of controlling mite infestation in a brood chamber comprising adapting a dosing gun to release a predetermined dosage of a parasiticide composition of the invention, wherein the predetermined dosage released is in an amount of 3 ml, each time the trigger is activated.

In an embodiment, the predetermined dosage of the parasiticide composition is ranging from 0.5 ml to 30 ml.

In an embodiment, the predetermined dosage of the parasiticide composition is ranging from 0.5 ml to 20 ml.

In an embodiment, the predetermined dosage of the parasiticide composition is ranging from 0.5 ml to 10 ml.

In an embodiment, the predetermined dosage of the parasiticide composition is ranging from 0.5 ml to 6 ml.

In an embodiment, the predetermined dosage of the parasiticide composition is ranging from 1 ml to 5 ml.

In an embodiment, the predetermined dosage of the composition is 3 ml.

In an embodiment, the present invention provides a method of controlling mite infestation in honeybee colonies in a brood chamber comprising using a dosing gun adapted to release a predetermined dosage of a semi-solid parasiticide composition comprising amitraz in an amount ranging from 10 mg to 110 mg per 3 ml of the composition in the brood chamber, such as in an amount ranging from 40 mg to 60 mg per 3 ml of the composition in the brood chamber.

In an embodiment, the present invention provides a method of controlling mite infestation in honeybee colonies in a brood chamber comprising using a dosing gun adapted to release a predetermined dosage of a semi-solid parasiticide composition comprising amitraz in an amount of 55 mg per 3 ml of the parasiticide gel composition in the brood chamber.

In an embodiment, the semi-solid composition may be applied directly using a dosing gun, a caulking gun.

The semi-solid composition may be applied on additional supports in a hive. The treatment is most effective if carried out over a complete brood cycle. The semi-solid compositions of the invention are effective for at least a 7 day period.

### Stabilisation of an octopamine receptor agonist

Another object of the invention is the use of a metal oxide for stabilizing an octopamine receptor agonist.

In some embodiments, the octopamine receptor agonist is one or more selected from the group consisting of amitraz or an octopamine receptor agonist of formula (I), as described above. Preferably, the octopamine receptor agonist is amitraz.

In some embodiments, the metal oxide is selected from (i) oxides of alkali metals, such as sodium oxide and potassium oxide or (ii) alkaline earth metals, such as calcium oxide, barium oxide and magnesium oxide. Preferably, the metal oxide is calcium oxide.

In some embodiments, the octopamine receptor agonist is amitraz and the metal oxide is calcium oxide

In some embodiments, the metal oxide is in an amount sufficient to stabilize the octopamine receptor agonist in the composition.

In some embodiments, the metal oxide is used for stabilizing an octopamine receptor agonist at a high temperature, such as at a temperature up to 100°C, for example at a temperature up to 140°C. This is particularly useful during an extrusion process wherein the temperature is increased above the fusion point of the composition to be extruded.

In some embodiments, the percentage of degradation of the octopamine receptor agonist is reduced and the total amount of the octopamine receptor agonist in the composition remains constant after at least twelve months of storage at room temperature.

All the features described herein may be combined with any of the above aspects, in any combination. In order that the present disclosure may be more readily understood, reference will now be made, by way of example, to the following description. However, the scope of the present disclosure is not limited by the examples in any manner. It will be appreciated by any person skilled in this art that the present disclosure includes aforesaid examples and further can be modified and altered within the scope of the present disclosure. It will be understood that all tests and physical properties listed have been determined at atmospheric pressure and room temperature (i.e. 25°C), unless otherwise stated herein, or unless otherwise stated in the referenced test methods and procedures.

It will be understood that the specification and examples are illustrative but not limitative of the present disclosure and that other embodiments within the spirit and scope of the invention will suggest themselves to those skilled in the art. Other embodiments can be practiced that are also within the scope of the present disclosure. The following examples illustrate the invention, but by no means intend to limit the scope of the claims.

### Brief description of the figures

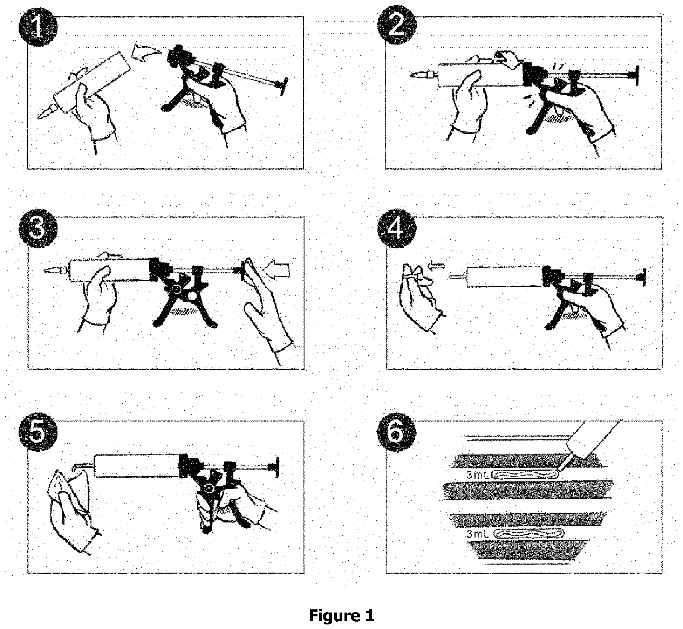

**Figure 1** depicts the stages of loading a dosing gun and its usage in loading the parasiticide composition of the present invention to a honey bee hive unit.

### EXAMPLES

### Example 1: Preparation of a semi-solid composition according to the invention

**Table 1 : semi-solid compositions**

| **No.** | **Ingredients** | **CAS number** | **Concentration % w/w** |
|---|---|---|---|
| 1. | Amitraz | 33089-61-1 | 2.00 |
| 2. | Calcium oxide | 1305-78-8 | 9.58 |
| 3. | Paraffin | 8009-03-8 | 88.42 |
| 4. | Total | | 100 |

**Process of preparation:** Paraffin was heated to 60°C using a water bath. The mixture was then stirred using a blade mixer. Once the mixture of paraffins was melted and homogeneous, amitraz was added under strong stirring. Calcium oxide was then stirred and mixed until completely dissolved and homogenized. The mixture was then heated at 45°C for 30 minutes before being packaged.

### Example 2: stability of a semi-solid composition according to the invention

**Table 2 : Amitraz semi-solid compositions +/- Calcium Oxide (CaO)**

| Raw material | Composition A % w/w | Composition B % w/w |
|---|---|---|
| Batch number | D20014 | D20015 |
| Amitraz | 1,33 | 1,25 |
| Liquid paraffin | Up to 100% | Up to 100% |
| Solid paraffin | 78,69 | 70,83 |
| CaO | - | 9,99 |

| | | |
|---|---|---|
| Note: Composition B is a semi-solid composition according to the present invention consisting in Amitraz, paraffin and calcium oxide. Composition A is a semi-solid composition comprising amitraz and paraffin. | | |

**Table 3 : Comparison of % degradation of Amitraz**

| **Composition** | **T0 % Amitraz** | **T1.5M % Amitraz** | **T3M % Amitraz** | **T6M % Amitraz** | **% degradation of Amitraz between T6M and T0** |
|---|---|---|---|---|---|
| Composition A | 100.00% | 99.09% | 96.36% | 95.45% | -4.55% |
| | CV: 0.3% | CV: 0.3% | CV: 0.4% | CV: 0.5% | |
| Composition B | 100.00% | 99.12% | 97.35% | 98.23% | -1.77% |
| | CV: 0.7% | CV: 0.7% | CV: 0.2% | CV: 1.8% | |

The above observations was made at 40°C/75%HR, the term T refers to the time interval at which the reading was made, the suffix refers to the month duration, i.e. at 1.5 months, 3 months and 6 months during storage. The term CV refers to the coefficient of variation between the different dosages realized for one interval.

It was observed from table 3 above that the composition of the present invention denoted as Composition B had the least amount of % degradation of amitraz, compared to Composition A which does not have the presence of calcium oxide.

### Example 3: device according to the invention and its use on hives Loading dosing gun

The parasiticide composition of Example 1 was loaded and secured into a dosing gun [as depicted in Figure 1 - 1, 2, 3]. The cap of the syringe was removed [Figure 1 - 4]. The trigger was gently and briefly depressed several times to prime tool ready for application of product to hives [Figure 1 - 5]. Each pull of the trigger delivered a standard dose of 3 ml of product. Accordingly, 2 doses of 3 ml were applied per brood chamber.

### Method of application

The parasiticide composition was used in brood free colonies as well as when the brood was present. The frames of the brood chamber were scraped clear of all wax and propolis that could prevent the bees from contacting the gel. The top feeders, top bars of the brood chamber, or for a double brood chamber hive, below the frames for the top brood chamber were cleaned. Two wooden supports were included per brood chamber on the top bars of frames in the centre of the brood area or in the centre of the bee cluster. For a better distribution of the active ingredient in the colony, a minimum of one frame free of composition was left between two frames with composition.

The dosing gun was used to apply 2 doses of 3 ml of the present parasiticide composition by applying a line of composition on each support. For a double brood chamber hive, the total dosage was 2 lines of 3 ml each in each brood chamber, i.e. a total of 12 ml of the composition was applied in the double brood chamber hive.

### Advantages of the parasiticide composition applied with a gun

The parasiticide composition reduces the load of phoretic mites with quick knockdown action.

The composition can be used any time when honey supers are not present and as the bees are active in the hives (avoid winter treatment).

In view of the quick action, the composition can be used several times during the year to control mite infestation.

### Example 4: Stability in an extruded strip

The following example shows the amitraz improvement stability by the use of Calcium Oxide in plastic extrusion process. This extrusion process was used to design specific non circular strip for in-hive use. The plastic polymer, the amitraz and the different excipients are mixed together and then extruded by heating to specific temperature conditions (up to 140°C) and then cooled down to obtain the strip shape.

**Table 4: solid compositions for extrusion**

| **Raw material** | **D19131** | **D19132** |
|---|---|---|
| Portaryte | 8,30% | 8,30% |
| Calcium oxide | 16,70% | - |
| Ethyl cellulose | - | 16,70% |
| Talc | 1,60% | 1,60% |
| Amitraz | 5,70% | 5,70% |
| EVA 1070 | 16.1% | 16.1% |
| EVA 2803G | Up to 100% | Up to 100% |

**Table 5 : Comparison of % degradation of Amitraz**

| Storage conditions | T0 % Amitraz | T1 month 40°C/75%HR % Amitraz | Deviation from T0 in % |
|---|---|---|---|
| D19131 | *100%* | *103.57%* | +3.57% |
| | | CV=0,1% | |
| D19132 | *100%* | *47.27%* | -52,73% |
| | | CV=0,3% | |

The amitraz stability after extrusion was drastically improved by the use of Calcium Oxide (D19131) after 1 month of storage at 40°C and 75% HR compared to the other composition without Calcium Oxide.

## Claims

1. Parasiticide composition for controlling mite infestation comprising:
(a) an octopamine receptor agonist, and
(b) a metal oxide.

2. Parasiticide composition according to any of the preceding claims, wherein the octopamine receptor agonist is amitraz or an octopamine receptor agonist of formula (I): 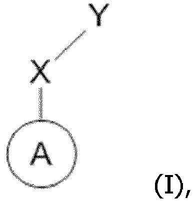 wherein:
A is selected from the group (II) or (III): 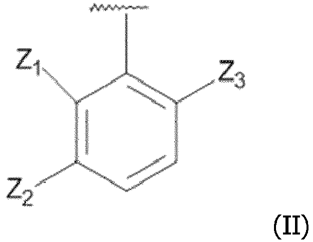 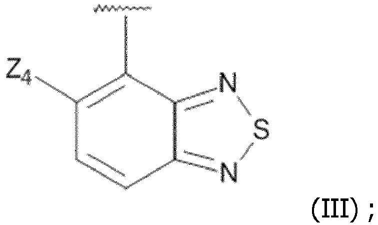
X is NH, CH2 or CH-CH3;
Y is a 5-bonded heterocycle comprising at least one nitrogen atom;
Z1 is a halogen, H or CH3;
Z2 is a halogen, H or CH3;
Z3 is a halogen, H or CH3;
Z4 is a halogen, H or CH3;
preferably the octopamine receptor agonist of formula (I) is detomidine (CAS number: 76631-46-4), medetomidine (D) (that is to say dexmedetomidine - CAS number: 113775-47-6), medetomidine (L) (that is to say levomedetomidine - CAS number: 119717-21-4), romifidine (CAS number: 65896-16-4), clonidine (CAS number: 4205-90-7), tizanidine (CAS number: 51322-75-9) or a mixture of two or more of these compounds.

3. Parasiticide composition according to any of the preceding claims, wherein the metal oxide is selected from (i) oxides of alkali metals, such as sodium oxide and potassium oxide or (ii) alkaline earth metals, such as calcium oxide, barium oxide and magnesium oxide.

4. Parasiticide composition according to any of the preceding claims, wherein the metal oxide is calcium oxide.

5. Parasiticide composition according to any of the preceding claims, wherein the octopamine receptor agonist is amitraz and the metal oxide is calcium oxide.

6. Parasiticide composition according to any of the preceding claims, wherein:
- the octopamine receptor agonist is in a concentration ranging from 0.1% to 20% w/w of the composition, such as from 0.5% to 10% w/w of the composition, such as from 1% to 5% w/w of the composition, such as from 1% to 3% w/w of the composition, such as 2% w/w of the composition, and/or
- the metal oxide is in a concentration ranging from 1% to 20% w/w of the composition, such as from 5% to 15% w/w of the composition, such as from 5% to 10% w/w of the composition, such as 9% w/w of the composition.

7. Parasiticide composition according to any of the preceding claims, wherein the composition further comprises one or more excipients.

8. Parasiticide composition according to any of the preceding claims, wherein the parasiticide composition of the invention is (i) in a liquid form; (ii) in a semi-solid form; or (iii) in a solid form.

9. Parasiticide composition according to any of the preceding claims, comprising:
- an effective amount of amitraz in a concentration ranging from 0.1% to 20% w/w of the composition, for example about 2.0% w/w of the composition;
- a stabilizing amount of calcium oxide in a concentration ranging from 1% to 20% w/w of the composition, for example about 9.5% w/w of the composition; and
- one or more excipients in a concentration ranging from 30% to 95% w/w of the composition, for example petrolatum, such as paraffin, in a concentration of about 88.5% w/w of the composition.

10. Composition according to any of claims 1 to 9 for use as a medicament.

11. Composition according to any of claims 1 to 9 for use as a parasiticide.

12. A device adapted for use in apiculture comprising a parasiticide composition according to any of claims 1 to 9, preferably the device is a strip, a brood chamber, a hive, a dosing gun.

13. Method for controlling mite infestation in honeybee colonies comprising exposing the honeybee colonies to a parasiticide composition according to any of claims 1 to 9.

14. Use of a parasiticide composition according to any of claims 1 to 9 for controlling mite infestation in honeybee colonies.

15. Use of a metal oxide for stabilizing an octopamine receptor agonist, preferably wherein the octopamine receptor agonist is amitraz and the metal oxide is calcium oxide.
